**Europäisches Patentamt**

**European Patent Office**

**Office européen des brevets**

(19)

(11) Veröffentlichungsnummer: **0 110 326**
A2

(12) # EUROPÄISCHE PATENTANMELDUNG

(21) Anmeldenummer: 83111742.9

(22) Anmeldetag: 24.11.83

(51) Int. Cl.³: **C 07 C 29/86,** C 07 C 31/10, C 07 C 31/12

(30) Priorität: 04.12.82 DE 3244958

(43) Veröffentlichungstag der Anmeldung: 13.06.84 Patentblatt 84/24

(84) Benannte Vertragsstaaten: **DE FR IT NL SE**

(71) Anmelder: **Wolff Walsrode Aktiengesellschaft, Postfach, D-3030 Walsrode 1 (DE)**

(72) Erfinder: **Hahn, Wolf-Diether, Dipl.-Chem., Beethovenstrasse 11, D-3030 Walsrode 1 (DE)**

(74) Vertreter: **Kutzenberger, Helga, Dr. et al, c/o Bayer AG Zentralbereich Patente Marken und Lizenzen, D-5090 Leverkusen 1, Bayerwerk (DE)**

(54) **Verfahren zum Entwässern von Alkoholen.**

(57) Verfahren zur Reduzierung des Wassergehaltes von niedrigen Alkoholen durch Extraktion mit Alkalilauge.

Wolff Walsrode AG

3030 Walsrode-Bomlitz
Str/m-c

## Verfahren zum Entwässern von Alkoholen

Die vorliegende Erfindung betrifft Verfahren zum weitgehenden Entwässern von niedrigen Alkoholen, insbesondere Alkoholen mit $C_3$-$C_4$, indem man den wasserhaltigen Alkohol mit Alkalihydroxid extrahiert.

Alkohole sind gängige Lösungsmittel in der chemischen Industrie, die aus ökonomischen Gründen wiederverwendet werden müssen. Da aber bei vielen Reaktionen Wasser in den Alkohol gelangt und viele Alkohole wie auch Propanole und Butanole mit Wasser azeotrope Gemische bilden können, müssen diese Alkohole vor ihrem Einsatz mit einem großen Kostenaufwand aufbereitet werden. Die übliche Aufbereitung ist eine zweistufige Destillation, wobei in der zweiten Stufe mit einem Schleppmittel - meistens Benzol - gearbeitet werden muß.

Überraschenderweise wurde nun gefunden, daß man die zweistufige Destillation vermeiden kann, indem man die wasserhaltigen Alkohole mit Alkalilauge extrahiert.

WW 5211

Gegenstand der Erfindung sind daher Verfahren zum Vermindern des Wassergehaltes, vorzugsweise $\leq$ 4 %, in Alkoholen, insbesondere in Alkoholen mit $C_3$-$C_4$, die dadurch gekennzeichnet sind, daß man den wasserhaltigen Alkohol in Gegenwart von wenigstens 20 Gew.-%, bezogen auf das Reaktionsgemisch, vorzugsweise 25 Gew.-%, ganz besonders bevorzugt mindestens 30 Gew.-%, Alkalihydroxid, extrahiert.

Nach dem erfindungsgemäßen Verfahren können alle niedrigen Alkohole entwässert werden, vorzugsweise Alkohole mit $C_3$-$C_4$, insbesondere Propanole und Butanole, die im System Alkohol, Alkali und Wasser eine ausreichend große Mischungslücke aufweisen.

Je nach Konzentration des Alkalihydroxids, vorzugsweise NaOH oder KOH, in dem System kann der Wassergehalt des Alkohols auf Werte unter 1 Gew.-% im Alkohol reduziert werden. Die Diagramme 1 bis 6 zeigen den Verlauf der Mischungslücken und zeigen, daß das Wasser im Alkohol durch Einstellung der Konzentration des Extraktionsmittels unter 1 Gew.-% reduziert werden kann.

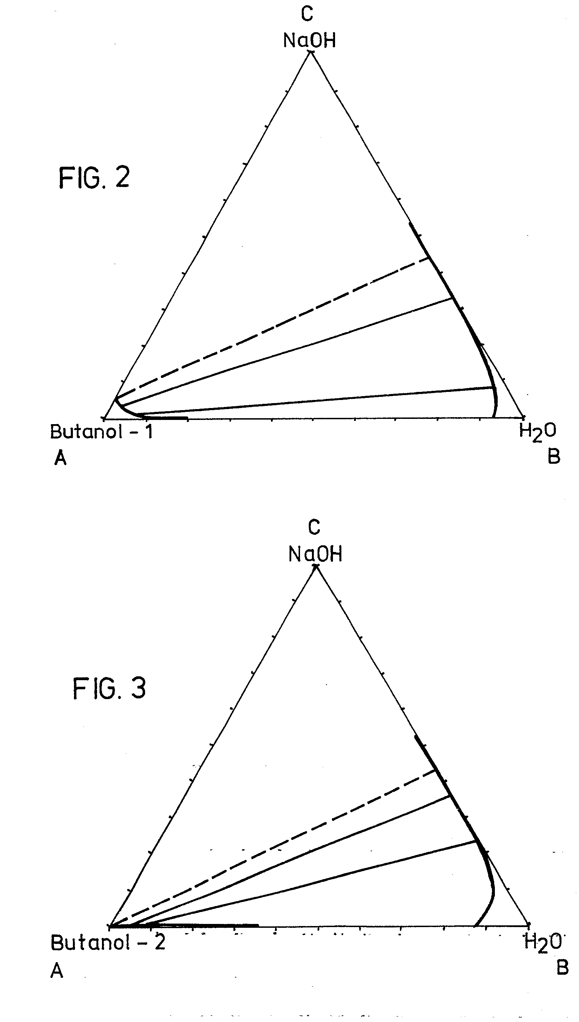

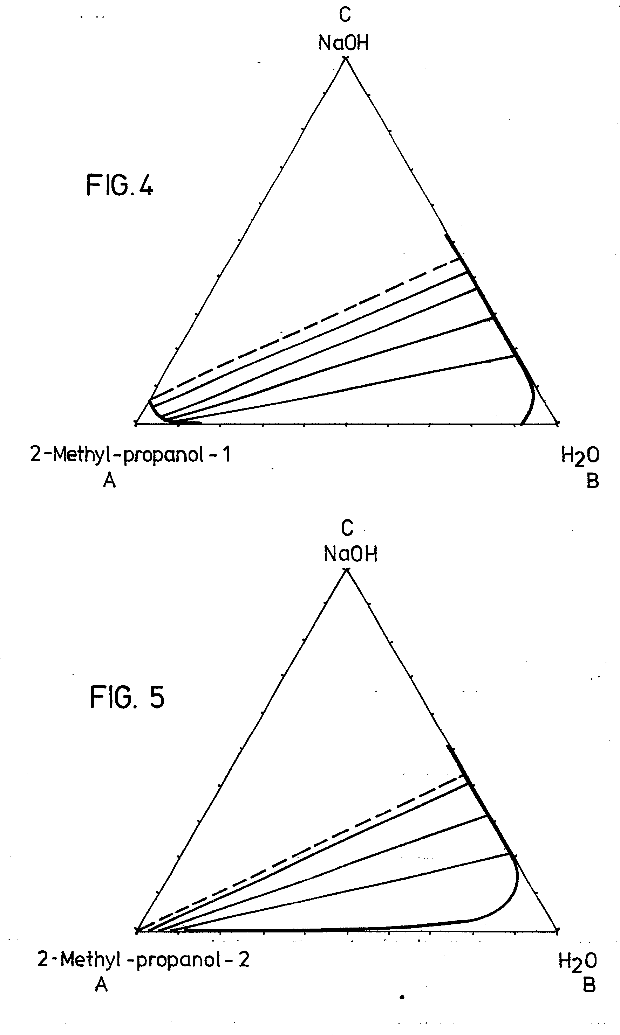

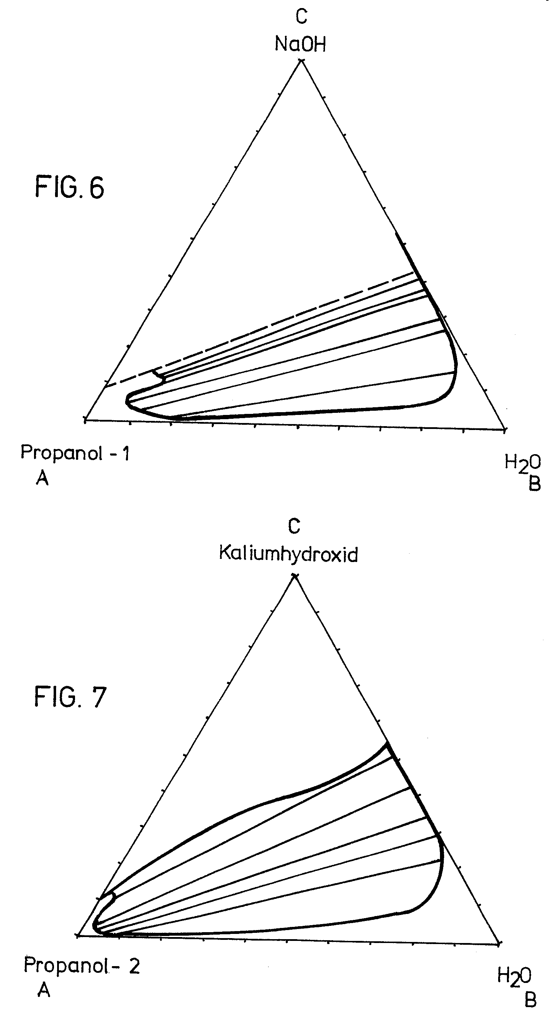

Die Fig. 2 zeigt die Binodalkurve des Systems Propanol-1/Wasser/Natriumhydroxid, wobei in jeder der Fig. 2 - 7 der Punkt A für 100 % Alkohol, der Punkt B für 100 % $H_2O$ und der Punkt C für 100 % Alkalihydroxid stehen.

Die Fig. 3 zeigt die Binodalkurve des Systems Butanol-2/Wasser/Natriumhydroxid.

Die Fig. 4 zeigt die Binodalkurve des Systems 2-Methyl-Propanol-1/Wasser/Natriumhydroxid.

<u>WW 5211</u>

0110326

Die Fig. 5 zeigt die Binodalkurve des Systems
2-Methyl-Propanol-2/Wasser/Natriumhydroxid.

Die Fig. 6 zeigt die Binodalkurve des Systems Propanol-1/Wasser/Natriumhydroxid.

Die Fig. 7 zeigt die Binodalkurve des Systems Propanol-2/Wasser/Kaliumhydroxid.

Die erfindungsgemäße Extraktion wird in bekannten Extraktionsapparaturen wie z.B. gepulste Siebbodenkolonnen,
Füllkörperkolonnen oder Rotating-disk-Contaktoren, entweder diskontinuierlich oder kontinuierlich durchgeführt.
Die kontinuierliche Verfahrensweise, insbesondere nach
dem Gegenstromprinzip, wird bevorzugt.

Bei dem erfindungsgemäßen Verfahren werden vorzugsweise
wäßrige Natron- bzw. Kalilauge eingesetzt, deren Konzentration beliebig variiert werden kann. Die Menge an
Alkalilauge, die bei der Extraktion zugegeben wird, richtet sich nach der Konzentration der Lauge und nach dem
gewünschten Grad der Entwässerung.

Die Reaktionstemperaturen liegen vorzugsweise im Bereich
von 20°C bis 10° unter dem Siedepunkt des zur Extraktion
gelangenden Systems, jedoch kann die Reaktion auch bei
Temperaturen von 20-25°C durchgeführt werden.

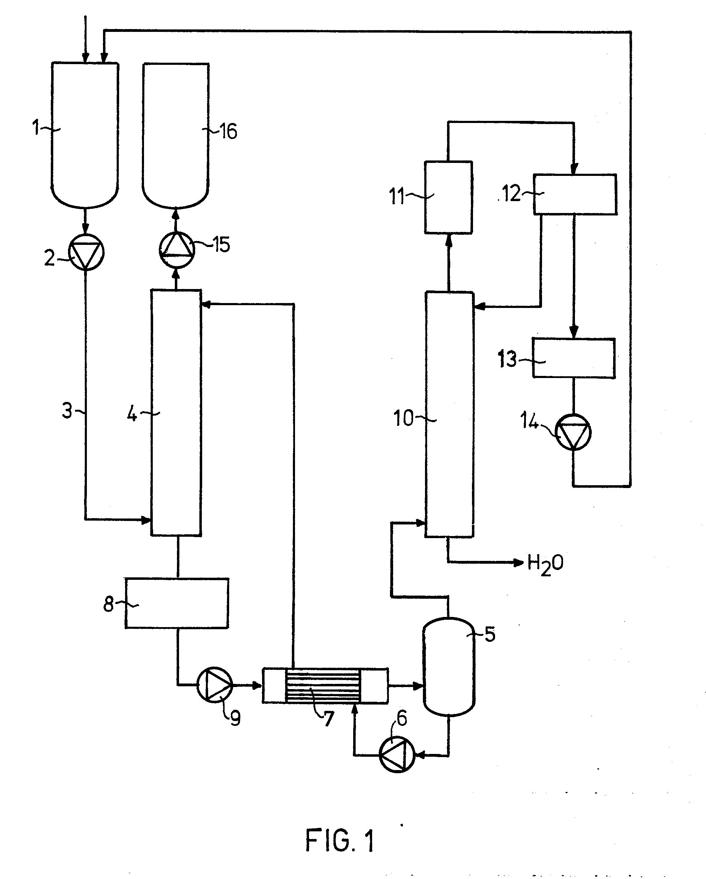

In Fig. 1 ist das erfindungsgemäße Verfahren schematisch
dargestellt. Es eignet sich insbesondere zur Reduktion
des Wassergehalts in Isopropylalkohol (IPA).

WW 5211

Aus dem Lagertank (1) wird wäßriges IPA mit einer Dosierpumpe (2) als konstanter Strom (3) in den Sumpf des Extraktionsapparates (4) eingespeist. Aus dem Eindampfer (5) fördert die Dosierpumpe (6) über einen Wärmeaustauscher (7) wäßrige Natronlauge bestimmter Konzentration auf den Kopf des Extraktionsapparates (4). In dem Extraktionsapparat (4) werden beide Ströme - der wäßrige IPA und die wäßrige Natronlauge - im Gegenstrom geführt, wobei es an sich gleichgültig ist, aus welchem Strom die zusammenhängende oder die disperse Phase gebildet wird.

Aus dem Kopf des Extraktionsapparates (4) tritt der entwässerte IPA, aus dem Sumpf eine verdünnte Natronlauge aus, die über eine Vorlage (8) mit der Dosierpumpe (9) über den Wärmetauscher (7) in den Eindampfer (5) zurückgegeben und in diesem wieder aufkonzentriert wird. Je nach dem Wassergehalt des wäßrigen IPA-Stromes (3) enthält die aus dem Extraktionsapparat (4) austretenden wäßrigen Natronlauge zwischen 1 bis 10 Gew.-% IPA, der zum größten Teil beim Aufkonzentrieren der umlaufenden Natronlauge im Eindampfer (5) zusammen mit dem Wasser als Brüden abgetrieben werden. Je nach absoluter Menge IPA in diesem Brüden kann dieser entweder verworfen oder in an sich bekannter Weise als wäßriger IPA zurückgewonnen werden, der wie in Figur 1 angegeben, dem Lagertank (1) wieder zugesetzt wird.

WW 5211

Die in Figur 1 beispielhaft dargestellte Rückgewinnung wird wie folgt durchgeführt:

Die aus dem Eindampfer (5) austretenden Brüden werden einer üblichen Destillierkolonne (10) zugeführt und durch den Kopfkondensator (11) kondensiert. Das Kondensat wird mit dem Rücklaufteiler (12) zum überwiegenden Teil wieder zurück auf den Kopf der Destillierkolonne (10), zum Teil aber auch in die IPA-Vorlage (13) gegeben, aus der die Pumpe (14) den zurückgewonnenen wäßrigen IPA in den Lagertank (1) zurückfördert. Das mitverdampfte Wasser wird dadurch kondensiert und läuft aus dem Sumpf in den Chemiekanal ab.

Im Falle der üblichen, bekannten Aufarbeitung durch Destillation hätte ein etwa 25 Gew.-% $H_2O$ enthaltendes IPA zunächst in Wasser und eine azeotrope IPA-Phase (ca. 12,6 Gew.-% $H_2O$) aufgearbeitet werden müssen, wobei letztere in einer zweiten Stufe durch Extraktivdestillation mit Benzol als Schleppmittel in IPA und eine IPA Wasser-Phase getrennt werden muß. Die IPA Wasser-Phase wird dann in das ursprüngliche Gemisch zurückgeführt.

WW 5211

## Beispiel 1

In einer gemäß Figur 1 gebauten Versuchsanlage, bei der der Extraktionsapparat eine mit Berl-Sätteln gefüllte Glaskolonne war, wurden 1.430 g/h eines wäßrigen IPA (30,0 Gew.-% $H_2O$) mit 226,1 g/h einer 45,15 Gew.-%igen NaOH im Gegenstrom extrahiert. Die Temperatur der aus dem Eindampfer 5 stammenden NaOH war 60°C.

Erhalten wurde ein IPA 1.000,5 g/h der Zusammensetzung 96,7 Gew.-% IPA; 3,0 Gew.-% $H_2O$ und 0,3 Gew.-% NaOH. Die ablaufenden Dünnlauge, 655 g/h enthielt neben 15 Gew.-% NaOH und 79,9 Gew.-% $H_2O$ noch 5,07 Gew.-% IPA, der aus den Brüden des Eindampfers 5 zu 90 % zurückgewonnen werden konnte.

## Beispiel 2

In derselben Versuchsanlage wie bei Beispiel 1 wurden 1.500 g/h eines wäßrigen Butanol-2 mit 35 Gew.-% $H_2O$ mit 444 g/h einer 42 Gew.-%igen wäßrigen NaOH extrahiert. Die Extrationstemperatur betrug 20 - 25°C (Raumtemperatur). Erhalten wurden 1.022 g/h eines Butanols mit 3,4 Gew.-% $H_2O$ und 0,1 NaOH. Die ablaufende Dünnlauge, 922 g/h, enthielt neben 20,0 Gew.-% NaOH und 78,8 Gew.-% $H_2O$ noch 1,2 Gew.-% Butanol-2, das zu ca. 75 % aus dem Brüden des Eindampfers 5 zurückgewonnen werden konnte.

0110326

**Beispiel 3**

In derselben Versuchsanlage wie in Beispiel 1 wurden 1.550 g/h einer wäßrigen 2-Methyl-Propanol-2 mit 40 Gew.-% $H_2O$ mit 593 g/h einer 42 Gew.-%igen wäßrigen NaOH bei Raumtemperatur extrahiert. Erhalten wurden 963 g/h eines 2-Methyl-Propanols-2 mit 4 Gew.-% $H_2O$ und 0,05 Gew.-% NaOH. Die ablaufende Dünnlauge (1.180 g/h) enthielt neben 21,2 Gew.-% NaOH und 78,3 Gew.-% $H_2O$ nur 0,5 Gew.-% 2-Methyl-Propanol-2, auf dessen Rückgewinnung aus den Brüden des Eindampfers 5 verzichtet wurde.

WW 5211

Patentanspruch:

1. Ein Verfahren zur Reduzierung des Wassergehaltes von niedrigen Alkoholen, dadurch gekennzeichnet, daß man den Alkohol in Gegenwart von Alkalihydroxid extrahiert.

2. Ein Verfahren nach Anspruch 1, dadurch gekennzeichnet, daß man Alkohole mit $C_3-C_4$ einsetzt.

3. Ein Verfahren nach Ansprüchen 1 oder 2, dadurch gekennzeichnet, das der wasserhaltige Alkohol in Gegenwart von wenigstens 20 Gew.-%, bezogen auf das Reaktionsgemisch, an Alkalihydroxid extrahiert wird.

4. Ein Verfahren nach Ansprüchen 1 - 3, dadurch gekennzeichnet, daß als Alkalihydroxid wäßrige Natron- oder Kalilauge verwendet wird.

5. Ein Verfahren nach Ansprüchen 1 - 4, dadurch gekennzeichnet, daß die Extraktion im Gegenstrom durchgeführt wird.

6. Ein Verfahren nach Ansprüchen 1 - 5, dadurch gekennzeichnet, daß aus einem Lagertank (1) wäßriger Alkohol mit einer Dosierpumpe (2) als konstanter Strom (3) in den Sumpf eines Extraktionsapparates (4) eingespeist, wäßrige Alkalilauge aus dem Eindampfer (5)

WW 5211

mit Hilfe der Dosierpumpe (6) über einen Wärmeaustauscher (7) auf den Kopf des Extraktionsapparates
(4) eingegeben wird und im Extraktionsapparat (4)
die beiden Ströme im Gegenstrom geführt werden, wobei aus dem Kopf des Extraktionsapparates (4) der
entwässerte Alkohol und aus dem Sumpf eine verdünnte Natronlauge austritt, die über eine Vorlage (8)
mit der Dosierpumpe (9) über den Wärmetauscher (7)
in den Eindampfer (5) zurückgegeben und in diesem
wieder aufkonzentriert wird.

WW 5211

FIG. 1

FIG. 2

C
NaOH

Butanol - 1
A

H₂O
B

FIG. 3

C
NaOH

Butanol - 2
A

H₂O
B

0110326
3/4

FIG. 4

C
NaOH

2-Methyl-propanol-1
A

H₂O
B

FIG. 5

C
NaOH

2-Methyl-propanol-2
A

H₂O
B

FIG. 6

C
NaOH

Propanol - 1
A

H₂O
B

FIG. 7

C
Kaliumhydroxid

Propanol- 2
A

H₂O
B